# EUROPÄISCHE PATENTSCHRIFT

(11) **EP 0 491 061 B1**
(45) Veröffentlichungstag und Bekanntmachung des Hinweises auf die Patenterteilung: **09.06.1999**
(21) Anmeldenummer: 90124374.1
(22) Anmeldetag: 17.12.1990
(51) Int. Cl.: A61K 31/40, A61K 38/55

(54) **Verwendung von N-(Aminoacyl)-aminosäuren zur Herstellung von dopaminergen und gonadotropen Arzneimitteln.**
Use of N-(aminoacyl)-amino acids for preparing dopaminergic and gonadotropic medicaments.
Utilisation d'acides aminés N-aminoacyle pour la preparation de médicaments dopaminergiques et gonadotropiques.

(43) Veröffentlichungstag der Anmeldung: 24.06.1992
(73) Patentinhaber: Weth, Gosbert Dr. med., Dr. rer. nat., 95346 Stadtsteinach (DE)
(72) Erfinder: Weth, Gosbert Dr. med., Dr. rer. nat., 95346 Stadtsteinach (DE)

(56) Entgegenhaltungen:
- EP-A- 0 180 785
- EP-A- 0 257 485
- Ikuo Saito et al.: ANGILOGY, Band 3, Nr. 3, 1989, Seiten 377-381
- JOURNAL OF BIOLOGICAL REGULATORS AND HOMEOSTATIC AGENTS, Band 3, Nr. 3, Juli-September 1989, Seiten 128-129, Milano, IT; C. LOMBARDI et al.: "Enalapril decreases plasma prolactin levels in hypertensive patients"
- MINERVA MEDICA, Band 81, Nr. 9, September 1990, Seiten 587-590, IT; C. LOMBARDI et al.: "Meccanismi di regolazione della secrezione di prolattina. Possibile ruolo del sistema renina-angiotensina ipofisario"
- J.E.F. REYNOLDS et al.: "Martindale, the Extra Pharmacopoeia, Ed. 29, 1989, the Pharmaceutical Press, Seiten 478-480, London, GB
- J.E.F. REYNOLDS et al.: "Matindale, the extra Pharmacopoeia, Ed. 29, 1989, the Pharmaceutical Press, Seiten 493, London, GB
- THE JOURNAL OF CLINICAL ENDOCRINOLOGY & METABOLISM, Band 64, Nr. 4, April 1987, Seiten 713-717, The Endocrine Society, US; W.B. MALARKEY et al.: "Angiote usin II promotes prolactin release from normal human anterior pituitary cell cultures in a calcium-dependent manner"

## Beschreibung

Die Erfindung betrifft die Verwendung von N-(Aminoacyl)-aminosäuren und deren Salze zur Herstellung von dopaminergen und gonadotropen pharmazeutischen Zubereitungen und Arzneimitteln.

Erhöhtes Noradrenalin ist mitverantwortlich für den Bluthochdruck. Es ist bekannt, daß ACE-Hemmer, bespielsweise Lopirin, therapeutisch in großem Ausmaß Anwendung finden. N-(Aminoacyl)-aminosäuren, wie Enalapril, senken bekanntlich den Blutdruck durch Hemmung der Bildung von Angiotensin II. Dabei wird auch eine leichte Reduktion des Noradrenalins diskutiert. Noradrenalin wird jedoch aus Dopamin gebildet, und somit wurde bisher angenommen, daß es neben der Bildung von Noradrenalin auch zu einer Senkung des Dopamins kommt.

Ferner ist bekannt, daß die Prolaktinfreisetzung durch den Hemmstoff PIF (Prolaktin-Inhibitor-Faktor), der als Dopamin identifiziert wurde, gehemmt wird (FORTH, HENSCHLER, RUMMEL, Bibliograph. Wissensch. Institut, "Allgemeine und spezielle Pharmakologie und Toxikologie, 1987, S. 124-139, S. 160 u. S. 400). Es ist bekannt, daß gonadotrope Hormone nur im Gehirn gebildet werden. Die gonadotrope Wirkung kann durch die bekannten gonadotropen Hormone FSH (Follikelstimulierendes Hormon) und LH (Luteotropes Hormon oder auch Luteinisierungs-Hormon genannt) festgestellt werden.

Diese Hormone stimulieren die Gonaden, steigern den Testosteronspiegel bei Männern und die Gestagene und Oestrogene bei Frauen.

Die FSH und LH produzierenden gonadotropen Zellen befinden sich in der Hypophyse (Funktion und Mechanismus in "Butt, W.R.: The chemistry of gonadotropins. Thomas, Springfield/III.1967).

Es wurde gefunden, daß N-(Aminoacyl)-aminosäuren durch Freisetzung von Dopamin zu einer peripheren und zerebralen Durchblutungssteigerung führen. Sie zeigen aufgrund ihrer dopaminergen und gonadotropen Wirkung einen stark durchblutungsfördernden Effekt. Es kommt zu einem hochsignifikaneten Anstieg von Dopamin bei gleichzeitiger Absenkung von Prolaktin, was bei dem bekannten Hintergrund überraschend ist.

Dopamin kann bei Durchblutungsstörungen, bei Patienten mit niedrigem Blutdruck zur Kreislaufstabilisierung eingesetzt werden. In Untersuchungen konnte gezeigt werden, daß es bei Verwendung von (N-Aminoacyl)-aminosäuren bei Patienten mit niedrigem Blutdruck, die hier für die Untersuchungen herangezogen werden, dagegen nicht zu einer weiteren Senkung des Blutdrucks kommt, sondern zu einer vermehrten peripheren und zentralen Durchblutung.

Dieser Effekt konnte cerebral nachgewiesen werden und die dopaminerge Wirkung konnte im Gehirn durch Neurotransmitterveränderungen nach der Methode von Dr. Dr. G. Weth, Dissertation, Würzburg 1987, S. 123-138, festgestellt und nachgewiesen werden. Untersuchungen an Patienten, die sublingual N-(Aminoacyl)-aminosäuren bekamen, zeigten zentral nachweisbare dopaminerge Wirkung.

In weiteren Untersuchungen konnte die gonadotrope Wirkung nachgewiesen werden, die ein weiterer Beweis für den zerebralen, durchblutungssteigernden Effekt ist.

Gemäß der Erfindung geeignete dopaminerge, gonadotrope, durchblutungsfördernde N-(Aminoacyl)-aminosäuren entsprechen der allgemeinen Formel 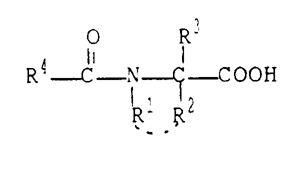 worin
- R¹: Wasserstoff, niedriges Alkyl oder Carbonyl,
- R²: Wasserstoff, niedriges Alkyl, Phenyl, Aralkyl, Imidazolylalkyl oder Indolylalkyl, die substituiert sein können durch niedriges Alkyl, Hydroxy, Alkylhydroxy, Methylendioxy, Mercapto, Alkylmercapto, Amino, Guanidino oder Carboxy,
- R¹ und R²: gemeinsam einen Pyrrolidin-, Piperidin- oder Thiazolidinring vervollständigen können, und die substituiert sein können durch ein niedriges Alkyl, Aralkyl, Phenyl, Furyl, Thienyl, Pyridyl oder Naphthyl, die ihrerseits substituiert sein können durch ein niedriges Alkyl, Hydroxyalkyl, Mercaptoalkyl, Hydoxy, Niedrigalkoxy, Alkylendioxy, Halogen, Nitro, Amino, Niedrigalkylamino oder Acylamino,
- R³: Wasserstoff oder Methyl und
- R⁴: Aminoalkyl, Amino, Amido oder Sulfid, die mit Substituenten entspechend R¹ und R² substituiert sein können,
und deren Salze.

Besonders geeignete N-(Aminoacyl)-aminosäuren sind (2R, 4R)-2-(2-Hydroxyphenyl)-3-(3-aminopropionyl)-4-thiazolidincarbonsäure und 1-[(2S)-3-Amino-2-alkyl-propionyl]-2-prolin, insb. 1-[(2S)-3-Amino-2-methylpropionyl]-2-prolin. Mit besonderem Vorteil werden im Handel erhältliche N-(Aminoacyl)-aminosäuren Enalaprilhydrogenmaleat (ein Derivat der N-(Carboxy)-aminosäure) und Perindopril verwendet.

Die erfindungsgemäß verwendeten Verbindungen eignen sich insbesondere als durchblutungsförderndes Medikament. Sie werden so mit Vorteil zur Herstellung von Arzneimitteln für die Behandlung von Krankheiten mit zerebraler und peripherer Durchblutungsminderung verwendet. Sie können als Tabletten, Granulat oder auch als Infusion hergestellt und verabreicht werden. Besondere Vorteile der erfindungsgemäß verwendeten Verbindungen in der medizinischen Behandlung liegen in der geringen Substanzbelastung des Patienten. Vielfach ist auch die Produktion der Mittel einfach und kostengünstig.

Die durch die erfindungsgemäß verwendeten N-(Aminoacyl)-aminosäuren erhöhte Freisetzung von Dopamin konnte durch pharmakologische Tests veranschaulicht und bewiesen werden. Die Dopaminbestimmung wurde nach G. Weth, "Das Verhalten der Neurotransmitter....", Dissertation, Würzburg, 1987, S. 123-138, durchgeführt.

Für die Untersuchungen wurden 2x12 Patienten (Alter 75±10 Jahre) herangezogen, die unter Durchblutungsstörungen, sowohl peripheren als auch zentralen, litten. Bei diesen Patienten wurde sowohl vor der Gabe von Enalaprilhydrogenmaleat, als auch 15, 30, 60, und 120 Minuten nach der Gabe von Enalaprilhydrogenmaleat Dopamin bestimmt. Dabei zeigte sich, daß es bei diesen Patienten nach 15 min zu einem hochsignifikanten Anstieg des Dopamins kam.

Die Patienten bekamen sublingual 20 ng Enalaprilhydrogenmaleat verabreicht (RR zwischen 100/70 und 170/110; MW 130/90 mm Hg).

Die Bestimmung der Dopaminwerte wurde wie folgt vorgenommen. In Zeitabschnitten von 15, 30, 60 und 120 Minuten wurden dem Patienten je 5 ml Blut entnommen und in speziell präparierten Vakuum-Behältern, die Glutathion und EDTA enthielten gegeben. Das Blut wird sofort auf 2°C abgekühlt und bei 2°C zentrifugiert. Das überstehende Blutplasma wurde bis zur Dopaminbestimmung bei minus 80 °C tiefgefroren.

Die Dimensionsangaben betragen für Dopamin pg/ml bzw. ng/l.

In den Tabellen sind die Ergebnisse der vorstehend aufgeführten pharmakologischen Untersuchungen zusammengestellt.

Tabelle 1 zeigt die statistische Auswertung nach Gabe von 20 mg Enalaprilhydrogenmaleat.

**Tabelle 1**

| Dopamin | Zeit | MW (pg/ml) | Str. | N | P (T) |
|---|---|---|---|---|---|
| Dop. | 0 | 105 | 67 | 12 | p<0,05 |
| Dop. | 15 | 143 | 78 | 12 | |
| Dop. | 0 | 105 | 67 | 12 | p<0,001 |
| Dop. | 30 | 188 | 72 | 12 | |
| Dop. | 0 | 105 | 67 | 12 | p<0,001 |
| Dop. | 60 | 253 | 81 | 12 | |
| Dop. | 0 | 105 | 67 | 12 | p<0,001 |
| Dop. | 90 | 230 | 62 | 12 | |
| Dop. | 0 | 105 | 67 | 12 | p<0,001 |
| Dop. | 120 | 182 | 53 | 12 | |
| (MW=Mittelwert, Str.=Streuung, N=Anzahl der Patienten, P(T)=Signifikanz für p<0,05). | | | | | |

Es ist hieraus ersichtlich, daß es 30 min nach Verabreichung von 20 mg der erfindungsgemäß verwendeten N-(Aminoacyl)-aminosäure zu einem hochsignifikanten Anstieg von Dopamin kommt.

Tabelle 2 zeigt die statistische Auswertung der gonadotropen Wirkung nach Gabe von 12 mg einer N-(Carboxy)-aminosäure.

Die statistische Auswertung der gonadotropen Wirkung, die durch die Bestimmung der Hypophysenhormone FSH (Follikelstimulierendes Hormon) und LH (Luteotropes Hormon) möglich ist, ergibt, daß es 30 min nach Verabreichung von 12 mg dieser Substanz zu einem signifikanten Anstieg von FSH und LH kommt.

Die Bestimmung von FSH und LH wurde radioimmunologisch mit Hilfe eines Doppelantikörpers, der mit 125-Jod markiert war (Firma Serono), bestimmt.Die Blutabnahme wurde vorgenommen wie oben beschrieben. Zu den angegebenen Zeiten wurde bei den Probanden je 5 ml Blut entnommen.

**Tabelle 2**

| Gonadotropes Hormon | Zeit | MW (ng/ml) | Str. | N | P(T) |
|---|---|---|---|---|---|
| LH | 0 | 6,4 | 3,1 | 12 | p<0,05 |
| LH | 30 | 9,2 | 3,0 | 12 | |
| FSH | 0 | 6,9 | 4,9 | 12 | p<0,001 |
| FSH | 30 | 7,9 | 4,4 | 12 | |
| (MW=Mittelwert, Str.=Streuung, N=Anzahl der Patienten, P(T)=Signifikanz für p<0,05). | | | | | |

Nachstehend wird die Erfindung anhand der Zeichnung näher erläutert:

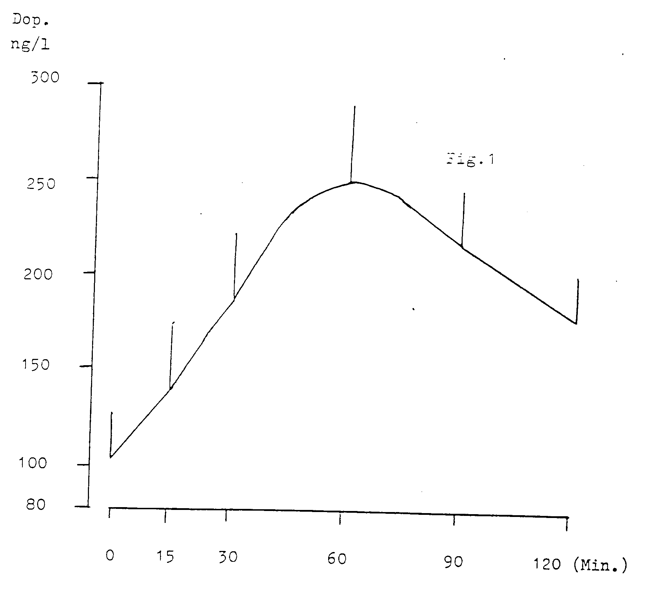

Fig. 1 zeigt den zeitlichen Verlauf der Dopaminfreisetzung nach sublingualer Gabe einer N-(Aminoacyl)-aminosäure. Auf der Abszisse ist die Zeit in Minuten und auf der Ordinate der Dopamingehalt im Plasma in ng/l aufgetragen. Sie zeigt, daß bei Gabe von 20 mg Enalaprilhydrogenmaleat der Patient (Durchschnittsgewicht 60kg) bereits nach 15 Minuten einen signifikanten Anstieg des Dopamins aufweist, der nach 60 min seinen Höhepunkt erreicht und dann wieder kontinuierlich abfällt.

## Patentansprüche

1. Verwendung von N-(Aminoacyl)-aminosäuren und deren Salzen zur Herstellung von dopaminergen und gonadotropen, pharmazeutischen Zubereitungen und Arzneimitteln.

2. Verwendung von N-(Aminoacyl)-aminosäuren und deren Salzen nach Anspruch 1 zur Herstellung von Arzneistoffen für die Behandlung von Krankheiten mit zerebraler und peripherer Durchblutungsminderung.

3. Verwendung von (2R,4R)-2-(2-Hydroxyphenyl)-3-(3-aminopropionyl)-4-thiazolidincarbonsäure nach Anspruch 1 und 2.

4. Verwendung von 1-[(2S)-3-Amino-2-alkylpropionyl]-2-prolin nach Anspruch 1 und 2.

5. Verwendung von 1-[(2S)-3-Amino-2-methylpropionyl]-2-prolin nach Anspruch 4.

6. Verwendung von Enalaprilhydrogenmaleat nach Anspruch 1 und 2.

## Claims

1. The use of N-(aminoacyl)-amino acids and their salts for the manufacturing of doaminergic, prolactin-lowering, gonadotropic pharmaceutical preparations and drugs.

2. The use of N-(aminoacyl)-amino acids and their salts as claimed in claim 1 for the manufacturing of drugs for the treatment of illnesses involving a decrease in cerebral and perpheral blood flow.

3. The use of (2R,4R)-2-(2-hydrocyphenyl)-3-(3-aminopropionyl)-4-thiazolidine carbonic acid as claimed in claims 1 and 2.

4. The use of 1[(2S)-3-amino-2-alkyl-propionyl]-2-proline as claimed in claims 1 and 2.

5. The use of 1-[(2S)-3-amino-2-methylpropionyl]-2-proline as claimed in claim 4.

6. The use of N-(carboxy)-amino acids as claimed in claims 1 and 2.

## Revendications

1. Utilisation de N-(aminoacyl)-acides aminés et de leurs pour la production de préparations pharmaceutiques dopaminergiques et gonadotropiques et de médicaments.

2. Utilisation de N-(aminoacyl)-acides aminés et de leurs sels conformément á la revendication 1 pour le traitement de maladies avec insuffisance vasomotrice périphérique et cérébrale.

3. Utilisation de (2R,4R)-2-(2-hydroxyphényle)-3-(3-mercaptopropionyle)-4-acides carboxyliques dé thiazolidine conformément à la revendication 1 et 2.

4. Utilisation de 1-[(2S)-3-Mercapto-2méthyle-propionyl]-2-proline conformément à la revendication 1 et 2.

5. Utilisation de 1[(2S)-3-Amino-2 methylpropionyl]-2-prolin conformént à la revendication 4.

6. Utilisation de Enalaprilhydrogenmaleat vonformément à la revendication 1 et 2.
